# EUROPEAN PATENT APPLICATION

(11) **EP 3 138 503 A1**
(43) Date of publication of application: **08.03.2017**
(21) Application number: 16186699.1
(22) Date of filing: 31.08.2016
(51) Int. Cl.: A61B 10/02, A61B 10/04

(54) **NEEDLE AND STYLET MANAGEMENT DEVICE**

(30) Priority: 31.08.2015 US 201562212028 P
(71) Applicant: Cook Medical Technologies LLC, Bloomington, IN 47404 (US)
(72) Inventor: O'CALLAGHAN, Rory, Co. Cavan (IE)
(74) Representative: Garratt, Peter Douglas

(57) **Abstract**

A stylet retraction system, the stylet retraction system comprising a stylet extending from a reel, where the reel is configured to move in a manner effecting longitudinal position change of the stylet along a longitudinal axis of the stylet, a needle at least partially surrounding the stylet, and a handle configured to effect longitudinal movement of the needle along a longitudinal axis of the needle, wherein the handle is rotatable with respect to the reel, and wherein an axis of rotation of the handle and an axis of rotation of the reel each is not coaxial with respect to the longitudinal axis of the stylet nor with respect to the longitudinal axis of the needle.

## Description

### CROSS-REFERENCE TO RELATED APPLICATIONS

This application claims priority to U.S. provisional application Serial No. 62/212,028, filed August 31, 2015.

### TECHNICAL FIELD

Embodiments disclosed herein generally relate to medical endoscopy devices. More particularly the embodiments relate to a needle system within which a needle and/or stylet is operable by rotation of a reel that rotates around an axis different than (including transverse to) the longitudinal axis of the needle and/or stylet.

### BACKGROUND

Fine needle aspiration (FNA) and fine needle biopsy are diagnostic biopsy procedures used to obtain a sample from a target site or area in a patient body. A fine needle (e.g., 19-gauge to 25-gauge) is directed to a target site, and suction is applied to the proximal end of a lumen of the needle to aspirate cells through its distal end. The procedure typically is far less invasive than other biopsy techniques, whether performed percutaneously (e.g., to sample a suspected breast tumor or subcutaneous lesion) or endoscopically (e.g., to sample a suspected cholangiocarcinoma via a duodenoscope). Moreover, advances in endoscopic ultrasound (EUS) technology have helped physicians and patients by providing enhanced ability of a physician to visualize a biopsy needle to obtain a sample of material from a target site without requiring an open incision or use of large-bore needles and/or percutaneous trocars.

In order to provide desirable pushability and trackability for these small-bore sample-collection needles, and to prevent inadvertent (e.g., early and/or late) collection of tissue in one or more distal needle openings, a stylet is typically provided through the length of the needle lumen. After the distal end opening(s) of the needle is/are directed to a target location via a medical endoscope such as a bronchoscope, an EUS endoscope, a duodenoscope, or other minimally-invasive endoscope device, the stylet is withdrawn and a syringe or other modality is attached to the proximal needle end for generating vacuum through the needle lumen to facilitate sample collection by drawing sample material into the distal end opening(s) of the needle. Stylet-management may pose challenges during such procedures.

Specifically, traditional stylets are often nearly 2 meters in length and must be handled by an operator, such as a nurse, after removal. These stylets are not sterile after having been in a patient. Further, multiple passes may be taken by an endoscopist before removing the needle, and some endoscopists use a fanning technique to take a sample from several different areas of a target area. The fanning technique may be difficult to control with traditional arm movements, and therefore the precision of the movement of the needle and/or the stylet near the target area may be lacking. Continued maneuvering also tends to wear out the tip of the needle, reducing its effectiveness and decreasing the quality of the collected sample. The use of a traditional stylet can create a number of time consuming tasks including feeding the stylet into a cannula during sample retrieval in procedures that may require multiple introductions and retractions of a stylet from a cannula lumen (e.g., placing the stylet in the lumen for navigation then removing it to allow pulling a vacuum through the lumen with a syringe or other vacuum source).

Thus, it is desirable to provide a needle and stylet management system with a handle assembly that provides for controlled, effective, and ergonomic user-initiated movements to operate the system.

### BRIEF SUMMARY

In particular, and as addressed by the system provided below, it is desirable to provide a needle and stylet management system that enables a user independently and selectably to deploy, retract, and re-deploy a needle and/or stylet with no proximal-end exposure of the stylet outside of the system, where such non-exposure provides improved stylet management and reduces the risk of the stylet contacting a non-patient non-sterile surface that would mitigate against it being re-introduced into the patient in the course of a single procedure or single series of procedures. The disclosed embodiments are directed at a needle and stylet management device. The needle and stylet management device may include a stylet extending from a reel, where the reel is rotatable in a manner effecting longitudinal position change of the stylet along a longitudinal axis of the stylet, a needle at least partially surrounding the stylet, and a handle, the handle being rotatable to effect longitudinal movement of the needle along a longitudinal axis of the needle. The handle may be rotatable with respect to the reel, and an axis of rotation of the handle and an axis of rotation of the reel each may not be coaxial with respect to the longitudinal axis of the stylet nor with respect to the longitudinal axis of the needle.

In some embodiments, the needle and stylet management device may include a base in mechanical communication with the reel, where the needle and stylet management device have a first state where the reel is rotatable in at least two directions with respect to the base. In a second state, the reel is substantially prevented from rotating in at least one of the at least two directions by the engagement of a ratchet pawl with at least one tooth located on the reel.

The needle and stylet management device may also comprise a ratchet mechanism. The ratchet mechanism may have a lever with a cam, where the cam is configured to contact a cam follower when the lever is in an engaged position. The lever may be configured to occupy either the engaged position or an unengaged position, and the cam follower may not contact the cam when the lever is in the unengaged position.

In some embodiments, the reel comprises teeth, and the needle and stylet management device may have a ratchet pawl configured to communicate with the teeth to limit the rotation of the reel in at least one direction. Further, the handle may be configured to effect rotation of the reel in at least one state.

### BRIEF DESCRIPTION OF THE DRAWINGS

FIG. 1A a plan view of a needle and stylet management system.
FIG. 1B is a detail view the distal end of a needle and stylet management system showing a sheath, needle, and stylet.
FIG. 2A is a perspective view of proximal-end components of a needle and stylet management system.
FIG. 2B is a side, partially cutout view of proximal-end components of a needle and stylet management system, taken along line 2B-2B of FIG. 2A.
FIG. 3A is a front view of a reel for use in a needle and stylet management system.
FIG. 3B is a perspective view angled to view the back of a reel for use in a needle and stylet management system.
FIG. 4A is a front view of a handle for use in a needle and stylet management system.
FIGS. 4B-C are perspective views of a handle for use in a needle and stylet management system.
FIG. 5A is a front view of a base for use in a needle and stylet management system.
FIG. 5B is a perspective view angled to view the back of a base for use in a needle and stylet management system.
FIG. 6 is a perspective view of internal elements of a needle and stylet management device with a stylet retraction lever in an unengaged position.
FIG. 7 is a perspective view of internal elements of a needle and stylet management device with a stylet retraction lever in an engaged position.
FIG. 8 is a perspective view of a ratchet pawl coupled to a pawl holder.
FIG. 9A is an angled perspective view of the front of a stylet retraction lever for use in a needle and stylet management system.
FIG. 9B is an angled perspective view of the back of a stylet retraction lever for use in a needle and stylet management system.
FIG. 10A is a front view of the proximal end of a needle and stylet management system with the stylet retraction lever in an unengaged position.
FIGS. 10B-E are front views of a needle and stylet management system which comprise an enlarged view of the distal end of the needle and stylet management system to illustrate its operation while the ratchet mechanism is not active.
FIG. 11A is a front view of the proximal end of a needle and stylet management system with the stylet retraction lever in an engaged position.
FIGS. 11B-E are front views of a needle and stylet management system which comprise an enlarged view of the distal end of the needle and stylet management system to illustrate its operation while the ratchet mechanism is active.
FIGS. 12A is an illustration showing the use of ultrasound imaging during a medical procedure utilizing a traditional stylet management system.
FIG. 12B is an illustration showing the use of ultrasound imaging during a medical procedure utilizing a needle and stylet management system described herein.
FIGS. 13A and 13B show, respectively, a user-facing side of a device embodiment, and an opposite side of that same embodiment.
FIGS. 14-24 illustrate certain features of structure and methods of use of a device embodiment.

### DETAILED DESCRIPTION OF THE DRAWINGS AND EMBODIMENTS

Various embodiments are described below with reference to the drawings, in which like elements generally are referred to by like numerals. The relationships and function(s) of the various elements of the embodiments may better be understood by reference to the following detailed description. However, embodiments are not limited to those illustrated in the drawings. It should be understood that the drawings are not necessarily to scale (although some drawings may be to scale, and drawings derived from photographs and/or production drawings will be to scale), and in certain instances details may have been omitted that are not necessary for an understanding of embodiments disclosed herein, such as-for example-conventional fabrication and assembly.

The invention is defined by the claims, may be embodied in many different forms, and should not be construed as limited to the embodiments set forth herein; rather, these embodiments are provided so that this disclosure will be thorough and complete, and will fully convey enabling disclosure to those skilled in the art. As used in this specification and the claims, the singular forms "a," "an," and "the" include plural referents unless the context clearly dictates otherwise. The terms "proximal" and "distal" are used herein in the common usage sense where they refer respectively to a handle/doctor-end of a device or related object and a tool/patient-end of a device or related object. The term "about" when used with reference to any volume, dimension, proportion, or other quantitative value is intended to communicate a definite and identifiable value within the standard parameters that would be understood by one of skill in the art (equivalent to a medical device engineer with experience in the field of tissue devices and/or pressure/vacuum-exertion and monitoring devices), and should be interpreted to include at least any legal equivalents, minor but functionally-insignificant variants, and including at least mathematically significant figures.

The needle and stylet management system is herein described, which may be used for the performance of a sample collection technique (e.g., FNA, FNB, etc.). The needle and stylet management system may vary from other devices where a stylet is withdrawn in linear fashion by user movement along its longitudinal axis from the proximal end of a device with a needle to create suction for recovering a sample, for example as described by U.S. Patent Application Pub. No. 2014/0114255 A1 and U.S. Patent Application Pub. No. 2012/0253228 A1. The embodiments described herein generally allow for the performance of a collection technique through rotational movements of a handle and reel, which may be in mechanical communication, and may respectively effect the movement of a needle and stylet. These embodiments provide the advantage of allowing a single user to effect the movement of both the stylet and the needle at one time. For example, a single surgeon may operate the handle through rotary movements to actuate the needle and stylet together into and out of target tissue during an FNA or other medical procedure. By activating the ratchet mechanism, as is described in detail herein, the medical professional may then operate only the stylet and/or only the needle to collect or eject a sample tissue. This rotary motion may be completed with one hand only. Further, the operation by rotary motion as described herein may provide an operator with increased precision over traditional stylet systems, as rotational wrist movements are more precise and controllable than lateral arm or hand movements. As opposed to traditional stylet systems, which may require that a stylet is drawn proximally from the proximal-most end of the device and must be handled, the described embodiments allow winding of the stylet within a reel as it is removed, which may decrease handling effort and time and may prevent contamination of the proximal end of the stylet as the stylet is withdrawn from the patient and/or needle (i.e., by keeping the proximal stylet end enclosed within the device so as to prevent exposed to -and specifically not contacting- potential non-sterile surfaces outside of the patient).

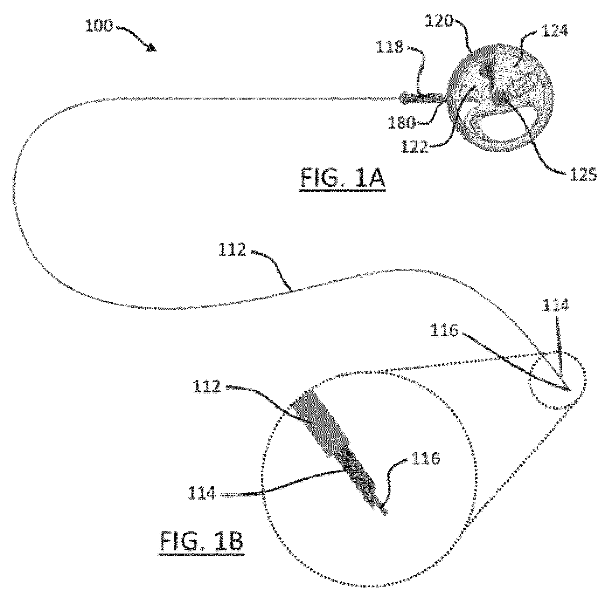

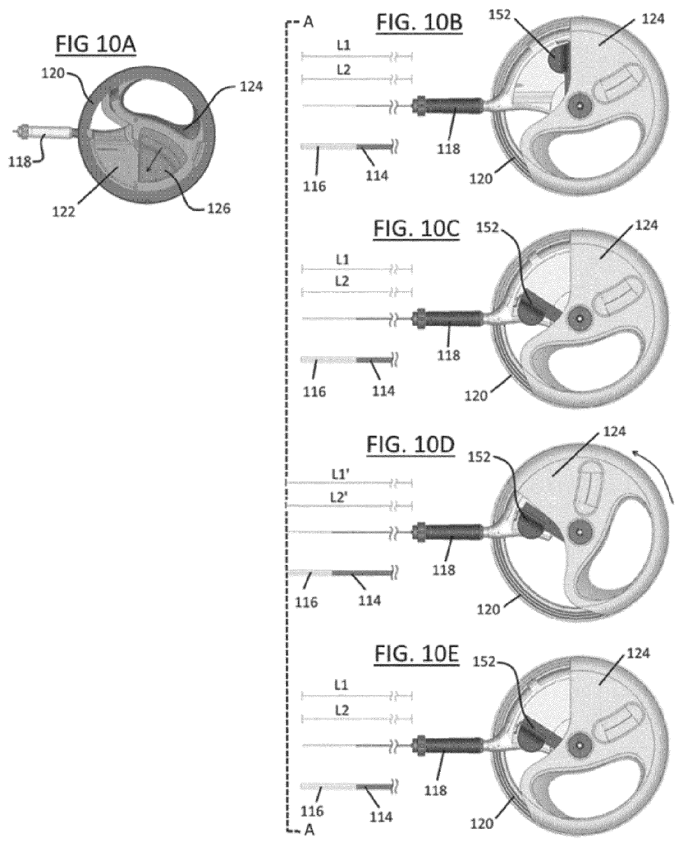

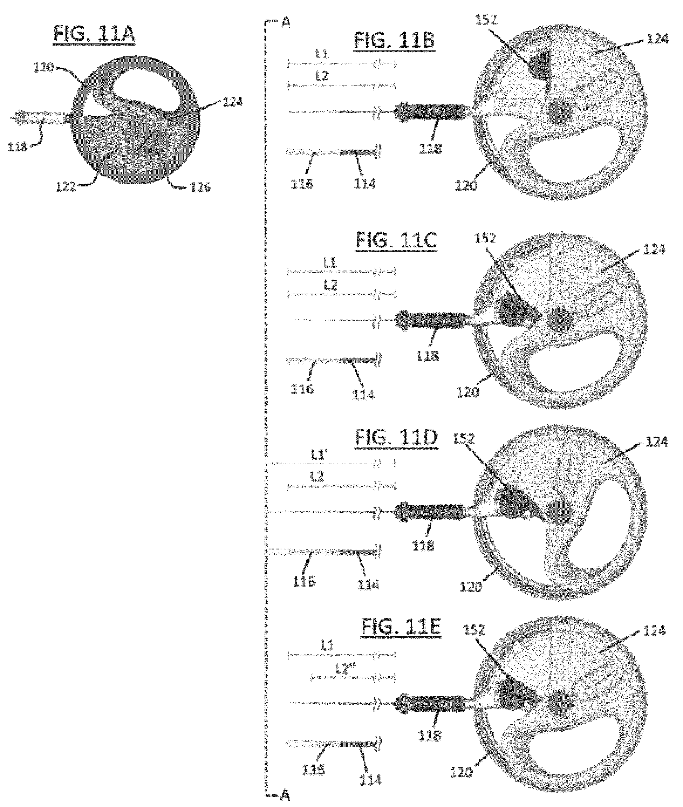

A needle and stylet management system 100 may be provided. Referring to FIGS. 1A-1B, the needle and stylet management system 100 may comprise a sheath 112 extending from an extender cap 118 at least partially surrounding a needle 114. The needle 114 at least partially surrounds a stylet 116. The stylet management system 100 may be configured to selectively move the needle 114 and/or the stylet 116. For example, as described in detail below, the stylet management system 100 may be capable of selectively effecting the longitudinal position of the needle 114 independent of the stylet 116 or effecting the longitudinal position of the needle 114 and the stylet 116 together (and both independent of the sheath 112). The sheath 112, needle 114, and stylet 116 may be configured to perform an FNA medical procedure, as described above, and may communicate with a stylet handling system 110 through an extender cap 118. As such, a user may rotatably actuate a handle 124 to move the needle 114 in reciprocating fashion and/or to extend or retract the stylet 116 relative to the needle 114. The extender cap 118 is attached directly to and extends from an extended portion 180 of a base 122 that protrudes outside a circle defined by handle 124 and reel 120. In the depicted embodiment, an axis of rotation (indeed, the main operative axis of rotation) of the handle 124 and an axis of rotation of the reel 120 each is not coaxial with respect to the longitudinal axis of the stylet 116 nor with respect to the longitudinal axis of the needle 114. This longitudinal axis of the stylet and of the needle is defined to describe the axis extending in the lengthwise direction of the stylet 116 and needle 114 in the region adjacent to the base 122 in the drawing figures (and particularly within the immediately adjacent region that is illustrated within the extender cap 118). The longitudinal axis of the stylet 116 retains this definition even in embodiments where the extender cap 118 is not present. At least a portion of the stylet 116 and the needle 114 may be coaxial with part of the base 122 and generally will be coaxial with the extended base portion 180. Those of skill in the art will appreciate that the rotary motion described with reference to the inventive embodiments herein accord more closely with the action of a distal endoscopic needle being actuated from the perspective as viewed via endoscopic ultrasound imaging (which is described below in greater detail with reference to FIGS. 10A-E and FIGS. 11A-E).

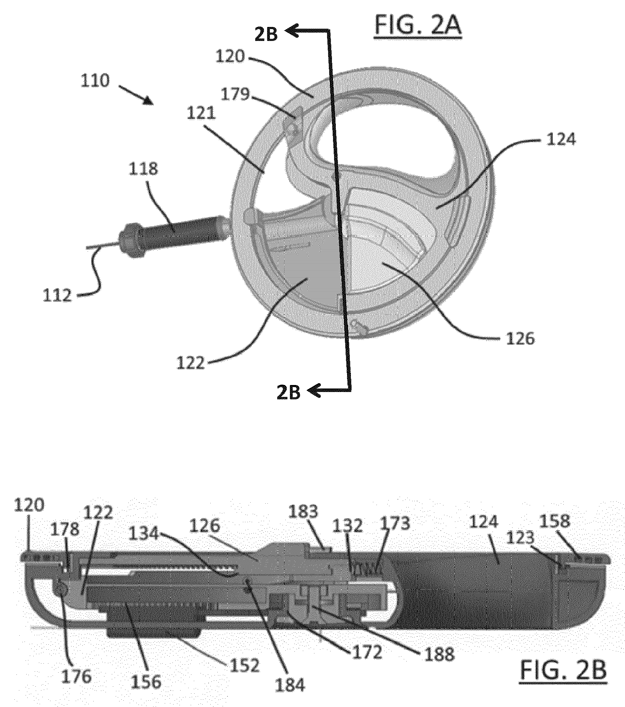

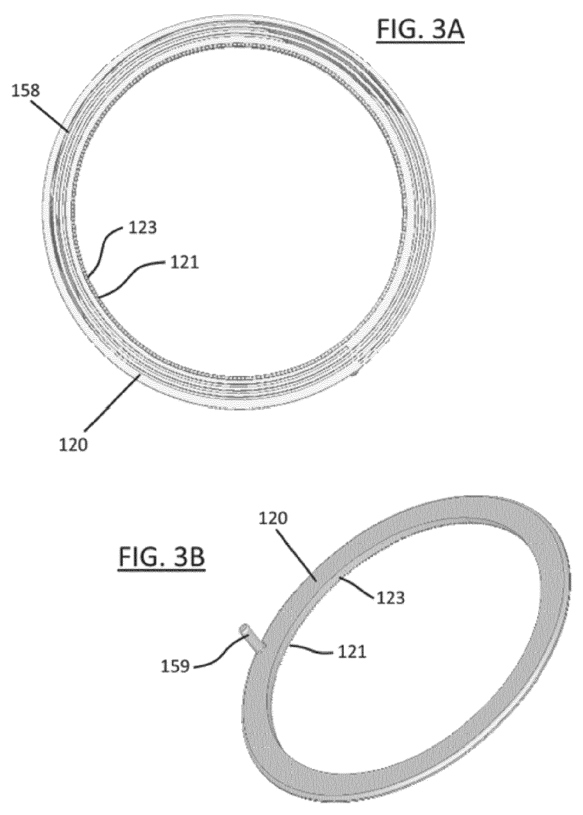

Referring to FIG. 2A, the stylet handling system 110 may comprise a handle 124 in mechanical communication with a stylet reel 120 having at least one tooth, depicted as teeth 121 (shown in more detail in FIG. 3). A base 122 may communicate with the reel 120 in a manner such that, as described herein, the reel 120 is selectively rotatable with respect to the base 122. The handle 124 may be rotatably coupled to the base 122 and may rotate around a central pivot 125 (shown in FIG. 1A). In the present embodiment, the handle 124 is attached to the needle 114 and the stylet reel 120 is attached to the stylet 116 such that the handle 124 and the stylet reel 120 effect the motion (and therefore longitudinal position) of the needle 114 and the stylet 116, respectively. The rotation of the handle 124, for example, may effect the longitudinal movement of the needle 114 along the needle's longitudinal axis. The reel 120, which is preferably in mechanical communication with the handle 124, may be rotatable, and the rotation of the reel 120 may effect the longitudinal movement of the stylet 116 along the stylet's longitudinal axis. The rotational axis of the reel 120 and the handle 124 may be orthogonal to at least one portion of the needle 114, the stylet 116, and/or the sheath 112, and potentially a portion of the needle 114, the stylet 116 and/or the sheath 112 is adjacent to, but distal of, the base 122, as depicted in FIG. 1A. Specifically, and with reference to the description above, the rotational axis of the reel 120 and the handle 124 will generally be orthogonal to at least the portion of the needle 114, the stylet 116, and/or the sheath 112 extending from and immediately adjacent to the protruding portion 180 of the base 122.

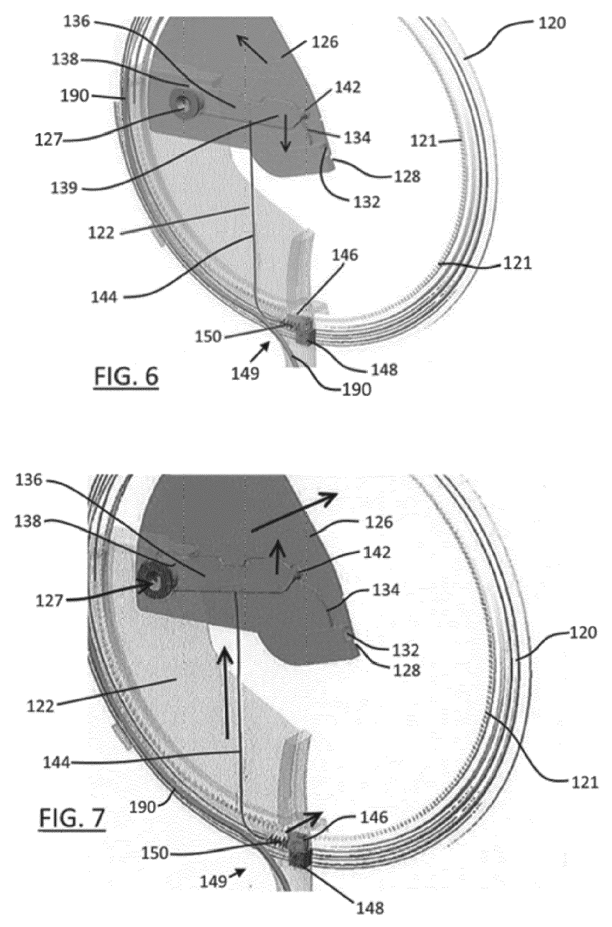

One embodiment of the reel 120 is shown in isolation in FIGS. 3A-B. The reel 120 generally has a curved shape in the form of a circle, oval, ellipse, etc. to facilitate rotation of the reel 120 when in mechanical communication with the handle 124 and/or the base 122 (see FIG. 2A). Referring to FIGS. 3A-B, the depicted embodiment of reel 120 is shaped as a ring and comprises teeth 121 on a projection 123 adjacent to the inner diameter of the reel 120. The reel 120 may comprise a spiral groove 158 configured to receive a stylet as it is drawn proximally into the device and may be coupled to the proximal end of the stylet 116, for example at an end of the spiral groove 158 nearest the outer edge of the reel 120. The stylet 116 may be attached to the reel 120 using an adhesive, a clamp, a knot, or any other suitable attachment means. During operation, the distal end/stylus of the stylet 116 may be directed into the spiral groove 158 via a stylet guide 190 (see FIG. 6), which may be coaxial to the stylet 116, may be flexible, and may be moveable (at least at its proximal end) to accommodate the spiral groove.

Further, the reel 120 may include a knob 159, which may be used to enhance the grip of a user, for example, when it is desired to rotate the reel 120 separate from other components (i.e., the handle 124). An embodiment with the knob 159 may be advantageous when it is desired to move the stylet 116 distally with respect to the needle 114 (for example, when ejecting a collected tissue sample from the needle 114 after completion of the collection procedure). In other embodiments, the reel 120 may comprise additional grooves or other suitable elements to guide the relative rotation of the reel 120 and handle 124 and the rotation of the reel 120 and/or handle 124 with respect to the base 122 (see FIG. 2A).

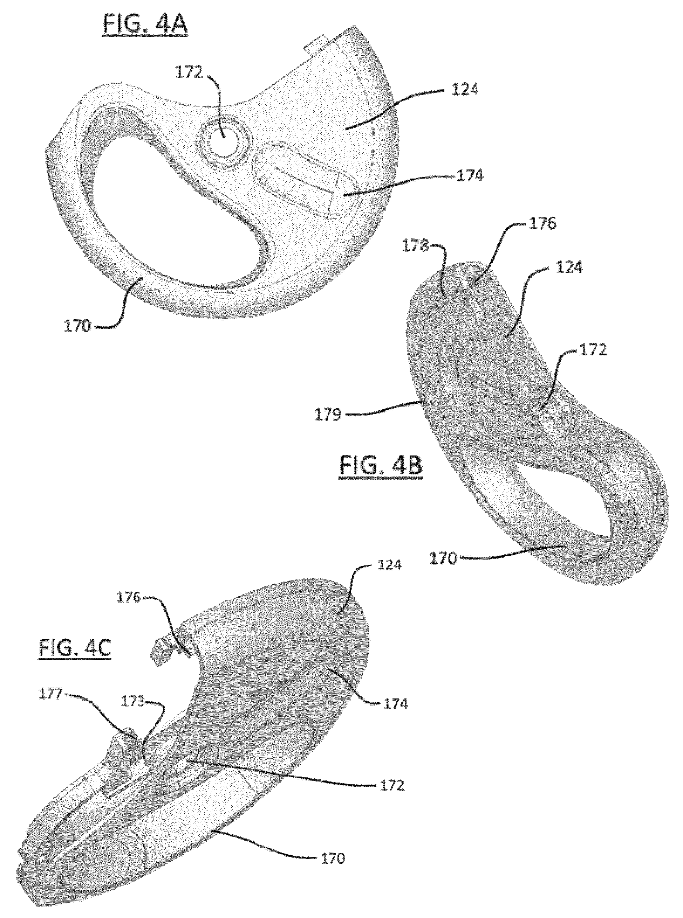

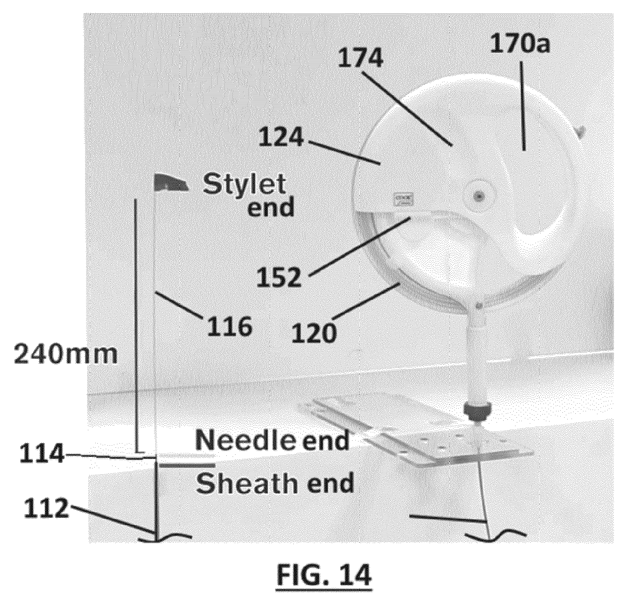

FIGS. 4A-C show an embodiment of the handle 124 in isolation. The handle 124 may comprise a hand portion 170 to provide a user with the ability to grasp, move, and/or rotate the handle 124. An indent 174 may further advance gripping ability by providing a surface for a user's thumb or fingertips to engage when his/her hand is placed through the aperture 170a shown as part of the hand portion 170 (see, e.g., the images and captions of FIGS. 14-24). The handle 124 may further comprise a central hole 172 configured to house the pivot 125 (see FIG. 1A) or otherwise facilitate the rotatable attachment of the handle 124 to the base 122 (see FIG. 2A). Referring to FIGS. 4B-C, the handle 124 comprises a recess 178 that is configured to communicate directly with the projection 123 of the reel 120 (see FIG. 3A). The projection 123, which may comprise the teeth 121, may extend parallel to the axis defined by the ring of reel 120 (shown in FIG. 3A), and may act as a track or guide for the rotation of the relative rotation between the reel 120 and the handle 124. The recess 178 may fit snugly over the projection 123 to provide a sufficient friction between the handle 124 and reel 120 such that the rotation of the handle 124 effects corresponding rotation of the reel 120. In some embodiments, the recess 178 is sized such that the teeth 121 to not make contact (e.g., to a surface) within the recess 178, which may protect the teeth 121 from wear.

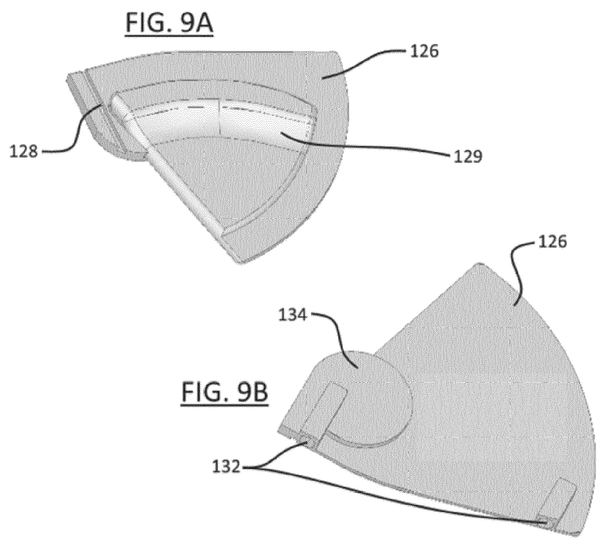

A tab 179 may be configured to contact a back surface of the reel 120 to keep the handle 124 and the reel 120, and particularly the projection 123 and the recess 178, in mechanical communication. A notch 177 may be included and configured to associate with a track 128 on a stylet retraction lever 126 (see FIG. 9B) to guide the movement of the stylet retraction lever 126, for example in a linear fashion. The handle 124 may comprise one or more spring mates 173 configured to communicate with a compression spring coupled to the stylet retraction lever 126, as described below. The handle 124 may also comprise a needle adaptor 176 configured to facilitate the attachment or other coupling of the above-described needle 114 (see FIG. 1B) to the handle 124. The needle adaptor 176 may couple to the proximal end of the needle 114 such that the movement of the needle adaptor 176 due to the rotation of the handle 124 effects proximal and distal movement of the needle 114. The needle 114 could otherwise be attached to the handle 124 using an adhesive, a clamp, or any other suitable device.

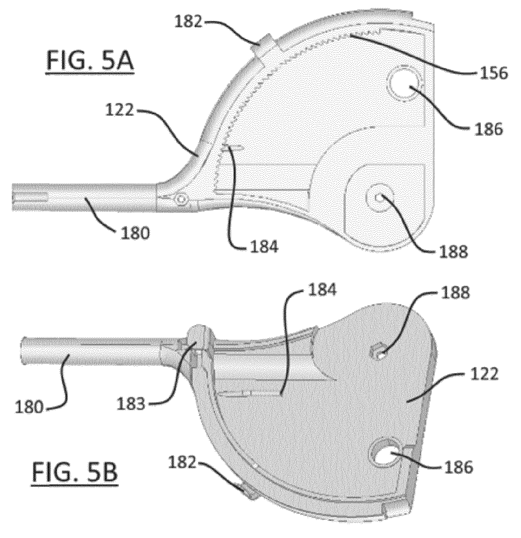

As described above, and referring to FIG. 2B, the handle 124 may be free to rotate around this central pivot 125 (shown in FIG. 1A, occupying central pivot hole 188 of FIG. 2B). The reel 120 may sit in the recess 178, potentially located near the outer diameter of the handle 124. As shown, the projection 123 extends into this recess 178 such that the two elements make contact, thereby keeping the reel 120 in communication with the handle 124 during use. The reel 120 may be prevented from falling out of the recess 178 through the utilization of at least one tab 183 located on the base 122 or handle 124 (*see also* FIG. 4B, tab 179, and FIG. 5B, tabs 182 and 183). The at least one tab 183 may be removable to allow insertion or removal of the reel 120 from the handle 124. The reel 120 may be rotatable with respect to the handle 124 due to smooth or lubricous contact surfaces, though in some embodiments it may be desirable to provide a slight amount of friction between the reel 120 and handle 124 such that the rotation of the handle 124 may effect rotation of the reel 120 when the ratchet mechanism 149 (described below with reference to FIGS. 6-7) is not activated. In some embodiments (e.g., those without significant friction between the reel 120 and handle 124), the rotation of the reel 120 may be achieved with the hand of an operator (e.g., the operator grips the handle 124 and reel 120 simultaneously) or through another suitable mechanism (e.g., a mechanism locking the reel 120 and handle 124 together).

FIGS. 5A-B show the base 122 in isolation. The base 122 may comprise teeth 156 which, as described in more detail below, may associate with a locking member for limiting the rotation of the handle 124 (see FIG. 2A) with respect to the base in at least one direction. The base 122 may further comprise an extended portion 180 with a lumen therethrough to provide a passage for the needle 114 and stylet 116 (see FIG. 1A). Tabs 182 and 183 are configured to associate with, for example, a surface or groove of the reel 120 (see FIGS. 3A-B) to facilitate the attachment and rotation of the reel 120 to the base 122. An eyelet 184 may be provided, as depicted, to allow a cable (such as cable 144 in FIGS. 6-7) or another suitable elongated element (e.g., a shaft, hydraulic device, etc.) to pass from one side of the base 122 to the other. A hole 186 may be provided to house a hinge or pivot 127 cooperating with a stylet retraction lever, as described below with reference to FIGS. 6-7. Further, a hole 188 may be provided to house the central pivot 125 (see FIG. 1), though the central pivot 125 may be provided without a hole 188 (e.g., when the pivot 125 is constructed as a boss integral with the base 122).

The handle 124 may comprise a stylet retraction lever 126, which is shown in greatest detail in FIGS. 6-7. The stylet retraction lever 126 may be selectively movable between an unengaged position (as depicted by FIG. 6) and an engaged position (as depicted by FIG. 7, where arrows are used to show the direction of relative bias or motion of certain components in FIG. 6 and the motion of certain elements in FIG. 7). When the stylet retraction lever 126 is in the unengaged position, a ratchet mechanism 149 is not active (or inactive). When the stylet retraction lever 126 is in the engaged position, the ratchet mechanism 149 is active. The stylet retraction lever 126 is shown in isolation in FIGS. 9A-B. For facilitation of desired movement of the stylet retraction lever 126, the stylet retraction lever 126 may comprise a track 128 configured to communicate with a notch 177 of the handle 124 (see FIG. 4C). At least one of spring holes 132 may be provided to at least partially house a compression spring (not shown), which may be used to create a tendency for the stylet retraction lever 126 to move into an unengaged position when no pressure is applied by a user. The compression spring may cooperate with a spring mate 173 located on the handle 124 (see FIG. 4C). In other words, the compression spring may operate such that the default state of the stylet retraction lever 126 is an unengaged state. The stylet retraction lever 126 may further comprise a cam 134 configured to communicate with a ratchet lever 136 (see FIGS. 6-7) that is configured to activate the ratchet mechanism 149. As described below, the cam 134 is preferably substantially circular in shape to facilitate proper communication with a cam follower 142 as the handle 124 rotates. A gripping surface 129 may be provided to facilitate gripping (and therefore selected movement) of the stylet retraction lever 126 by a user.

Referring back to FIGS. 6-7, in the depicted embodiment, the ratchet lever 136 comprises a first end 138 coupled to the base 122 at pivot 127. The ratchet lever 136 further comprises a second end 139 comprising the cam follower 142. The cam follower 142 is configured to communicate with the cam 134 when the stylet retraction lever 126 is in the engaged position. The stylet retraction lever 126 is not engaged (i.e., unengaged) when no pressure is applied by a user, and therefore the stylet retraction lever 126 is also in an unengaged position, as depicted by FIG. 6. In the depicted embodiment, the unengaged position corresponds with an outward position with respect to the stylet retraction lever 126. The influence outwards into the unengaged position may be provided by a compression spring, as described above. In the unengaged position, the stylet retraction lever 126 is sized such that the cam 134 does not communicate the ratchet lever 136 (see FIG. 6). Accordingly, when the stylet retraction lever 126 is not engaged, the handle 124 (see FIG. 2A) comprising the stylet retraction lever 126 may rotate without moving the ratchet lever 136. As described below, the engagement of the stylet retraction lever 126 activates the ratchet mechanism 149.

In FIG. 7, the stylet retraction lever 126 has been moved into an engaged position, which corresponds with an inward position in the depicted embodiment. In this position, the cam 134 has moved into communication with the cam follower 142, thereby providing an upward force on the ratchet lever 136, which, as described below, activates the ratchet mechanism 149 (see FIGS. 6-7). In the depicted embodiment, the cam 134 is substantially circular such that as the handle 124 rotates, the profile of the cam 134 at the location of the cam follower 142 will remain constant despite the rotation of the cam 134. In other words, when the stylet retraction lever 126 is in the engaged state, the cam 134 dynamically provides an upward force on the ratchet lever 136, thereby keeping the ratchet mechanism 149 engaged as the handle 124 (see FIG. 2A) rotates about pivot 125.

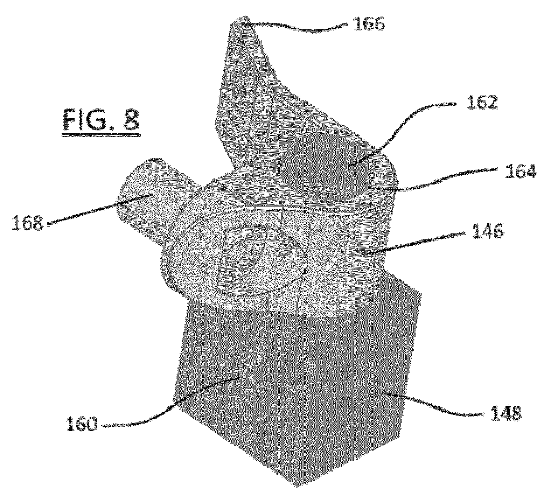

Referring to FIGS. 6-8, the ratchet lever 136 is connected to a cable 144 or another suitable connection member at its proximal end. The distal end of cable 144 may be attached to a ratchet pawl 146, and the ratchet pawl 146 may be coupled to a pawl holder 148. For illustration purposes, one embodiment of the ratchet pawl 146 is depicted in isolation in FIG. 8. The ratchet pawl 146 comprises a stop 166. The stop 166 is configured to selectively engage the teeth 121 of the reel 120 when, as described herein, the ratchet lever 136 is in an engaged position. In the depicted embodiment, the pawl holder 148 may be attached to or a part of the base 122. This attachment may be provided by utilizing, for example, a fastener configured to cooperate with bore 147, but any suitable attachment device may be used. The pawl holder 148 may comprise a cylindrical pin 162, which may correspond with a cylindrical bore 164 provided through the ratchet pawl 146. In this embodiment, the cylindrical pin 162 and bore 164 are configured such that the ratchet pawl 146 may rotate about the cylindrical pin 162, thereby moving the stop 166 into and out of engagement with the teeth 121. In some embodiments, the ratchet pawl 146 may comprise a pin 168 configured to communicate with a compression spring 150 to provide a default tendency for the ratchet pawl 146 to be positioned out of communication (i.e., out of contact) with teeth 121. Stop 166 preferably is angled with respect to teeth 121 such that, when in engagement with teeth 121, it allows motion of the stylet reel 120 in one direction only. In the described embodiment, the stop 166 is angled such that when engaged, the stylet reel 120 can rotate clockwise (thereby pulling stylet 116 distally), but cannot achieve counterclockwise rotation with respect to the base 122. In other embodiments, the limitation on rotation could be reversed.

FIGS. 10A-E illustrate the operation of the needle and stylet management system 100 when the stylet retraction lever 126 is not engaged. In FIG. 10A, the stylet retraction lever 126 is in the unengaged position, and therefore, as described above, the ratchet pawl 146 is out of contact or other communication with teeth 121 (see FIG. 4). For ease of explanation, a reverse/inverted view is shown in FIGS. 10B-10E. In FIG. 10B, handle 124 is in a depicted first handle position. A locking member 152 is in locked position. In the current embodiment, the locking member 152 comprises a locking member pawl 154 for engaging the teeth 156 located on the base 122. Other suitable locking devices can be used. The locking member 152 is configured to prevent the handle 124 from rotating with respect to the base 122 when the locking member 152 is in a locked position, as depicted by FIG. 10B. The locking member 152 may comprise a portion that extends into the path of rotation of the handle 124, thereby preventing rotation of handle 124. The locking member 152 may further comprise a tab or other device for facilitating gripping, which may be configured to grip or otherwise facilitate movement by a user.

Referring to FIG. 10C, the locking member 152 has been moved from a locking position (in FIG. 10B) to an unlocked position as depicted by FIG. 10C. It is noted that the base 122 may comprise a plurality of teeth 156 for allowing the locking member 152 to sit in any number of positions, thereby variably and selectively restrict the motion of the handle 124. Including a locking member may be advantageous, as it will allow a user to set the extent to which a handle may rotate. For example, a medical professional may desire to lock the handle in place when performing certain aspects of an FNA procedure where it is desired for the stylet and/or the needle to remain in place with respect to a target tissue.

As shown in FIG. 10B, the needle 114 and the stylet 116 are in a first state. In this state, a first length L1 may be associated with the needle 114. As depicted, the first length L1 corresponds with the length of the needle 114 to its terminal distal end from an extender cap 118, which is extends from the protruding portion 180 of the base 122. It is noted that the length L1 (and similarly other lengths referred to herein) do not correspond to the isolated span of the needle 114 (or stylet 116), but rather the length of its extension from the extender cap 118. This length is used to compare the position of the terminal end of the needle 114 for purposes of simple explanation. Similarly, there is a second length L2 that corresponds with the stylet 116. As depicted, the while the handle is in the first position, the stylet 116 and needle 114 extend coaxially equal lengths L1 and L2 relative to the extender cap 118. In some cases, the stylet 116 may initially extend more than (and therefore stick out of) the needle 114, or may extend less than the needle 114 in the first state.

Referring to FIG. 10D, the handle 124 has rotated in the counterclockwise direction to a second handle position. As a result, the distal terminal end of needle 114, (where the proximal terminal end is coupled to the handle 124), has moved a distal distance such that it now extends a length of L1' from the extender cap 118 to a position depicted at reference line A-A, which serves to illustrate the relative lengths described herein. Because the stylet retraction lever 126 was not engaged, the rotation of the handle 124 also caused movement of the stylet 116 in the distal direction due to the tendency of the stylet reel 120 to move with the handle 124. The distal terminal end of stylet 116 therefore moved distally with the needle 114, and the stylet 116 comprises a length L2' equal to the length L1'.

Referring to FIG. 10E, the handle 124 has rotated in the clockwise direction back to the first position. Here, the motion occurred still without engaging the retraction lever 126. Because the needle 114 is coupled to the handle 124, the handle 124 has moved the needle 114 proximally such the needle 114 again has a length L1. Because the stylet retraction lever 126 was not in the engaged position, the ratchet pawl 146 did not activate (i.e., engage with the teeth 121, see FIG. 7), and the stylet 116 was moved proximally by the clockwise motion of the handle 124. The movement of the handle 124 moved the stylet 116 with the needle 114 such that the stylet 116 again has a length of L2.

FIGS. 11A-E illustrate the operation of the needle and stylet management system 100 when the retraction lever 126 is engaged, as shown by FIG. 11A. For ease of explanation, a reverse/inverted view is shown in FIGS. 11B-11E. Moving the stylet retraction lever 126 engages the ratchet pawl with the teeth 121 of the stylet reel 120, as described above (see FIGS. 7-8). Referring to FIG. 11B, the handle 124 is in the first position, the needle 114 has a length of L1, and the stylet 116 has a length of L2. Here, the state of the needle and stylet management system 100 is identical to the state of FIG. 10B except that the retraction lever 126 is in the engaged position. In FIG. 11C, the locking member 152 has moved into the unlocked position.

Referring to FIG. 11D, the handle 124 has rotated in the counterclockwise direction to the second position. As a result, the needle 114, which is coupled to the handle 124, has moved a distance such that it now extends a length L1' (to reference line A-A) from the extender cap 118. Because the retraction lever 126 is engaged, the stylet reel 120 cannot rotate with respect to the base 122, and therefore the stylet 116 does not move distally. Here, the needle 114 extends a length of L1', while the stylet 116 retains its previous length of L2. As a result, in this embodiment, the terminal end of the stylet 116 has been drawn into the lumen of the needle 114.

As shown in FIG. 11E, the handle 124, with the stylet retraction lever 126 still engaged, has moved in the clockwise direction back to the first position. Because the needle 114 is coupled to the handle 124, the handle 124 has moved the needle 114 proximally such the needle 114 again has a length L1. While the retraction lever 126 is engaged, the angled ratchet pawl 146 allows the stylet reel 120 to move clockwise, and therefore the clockwise movement of the handle 124 moved the stylet reel 120 and the coupled stylet 116 in the proximal direction. Both the needle 114 and the stylet 116 moved proximally. The stylet 116 has a new length of L2", which is shorter than its original length of L2. In the depicted embodiment, the terminal end of stylet 116 has remained drawn within the needle 114.

The procedure from FIG. 11A to FIG. 11D can be repeated. So long as the stylet retraction lever 126 is engaged, each counterclockwise motion of the handle 124 with draw the terminal end of stylet 116 further within the lumen of the needle 114 (though, as noted above, the draw occurs when the needle moves distally while the stylet 116 remains in position). Each clockwise motion of the handle 124 will pull both the needle 114 and the stylet 116 distally.

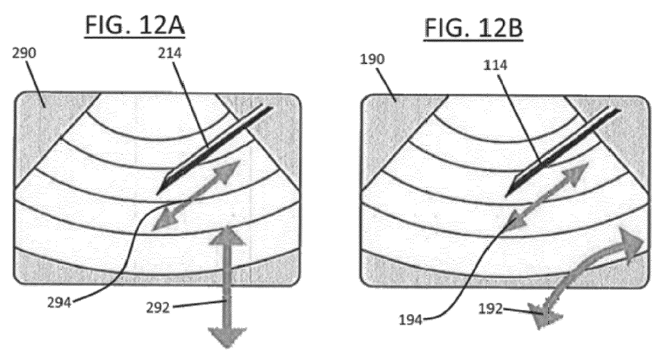

Advantageously, the rotary motion described with reference to operation of the inventive embodiments herein will accord more closely with the action of a distal endoscopic needle being actuated from the perspective as viewed via endoscopic ultrasound imaging. Referring to FIGS. 12A, a screen 290 is configured for use during endoscopic ultrasound imaging. Those skilled in the art will appreciate that, in traditional stylet systems where operation is achieved by withdrawing a needle in the longitudinal direction at the proximal end of the system, the vertical actuation or movement (depicted as movement 292) of the needle handle may correspond to visual feedback on screen 290 such that the direction of movement of the needle 214 is substantially different than the direction of user-initiated movement 292. This may increase the difficulty of precisely moving and targeting the needle 214 to a specific location within the body, or at least make it less intuitive. The difficulty may be enhanced during complex medical procedures requiring multi-tasking and tasks potentially performed by more than one user (e.g., a doctor and a nurse), as is common when using a traditional needle and stylet management system. Further, because the screen 290 may depict a zoomed-in view of the needle 214 within the body, the ratio of the handle actuation or movement 292 to the movement of the needle 214 may not be 1:1. In other words, a small amount of movement 292 may correspond with a large movement 294 as displayed on the screen, further increasing the difficulty of needle placement.

In the embodiments of the needle and stylet management system described herein, a user will find that these difficulties are alleviated. Referring to FIG. 12B, a needle 114 may be located near the distal end of a needle and stylet management system as described herein. The rotational movement 192 of the handle 124 and/or reel 120 may accord more closely with the movement 194 as depicted on the screen from the perspective of the user. Additionally, the ability for the user to use precise rotational wrist movements (as opposed to less precise linear arm movements) will allow the user to better transpose the movement 194 of a handle and/or reel with a scaled screen perspective corresponding to movement 192. The potential for single-user operation with ability to both move the needle 114 and withdrawn a stylet as described herein may further reduce the complexity and difficulty of utilizing ultrasound imaging during a sample collection procedure, such as a diagnostic biopsy procedure.

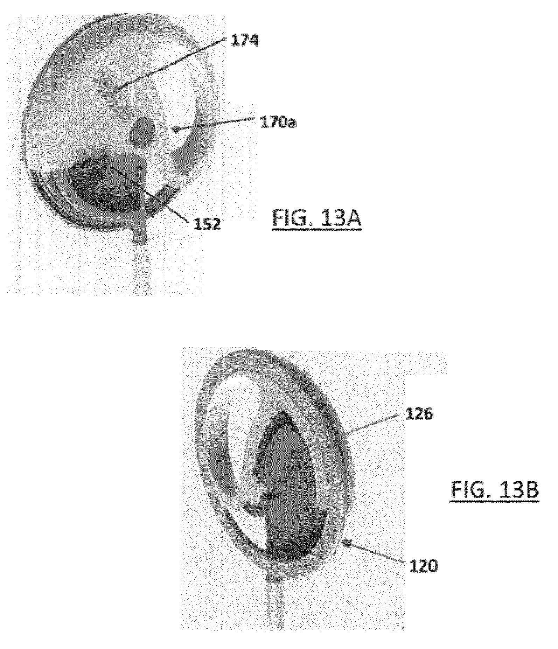

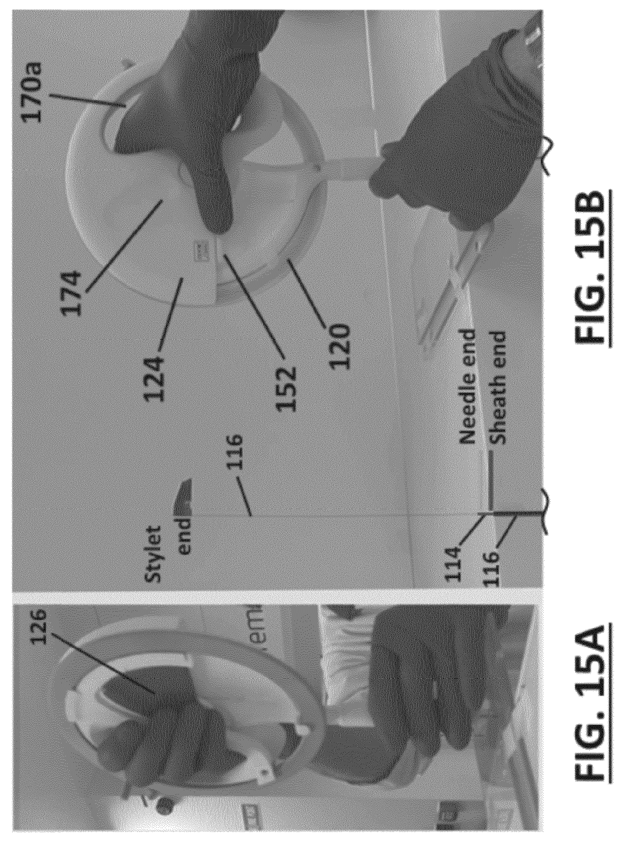

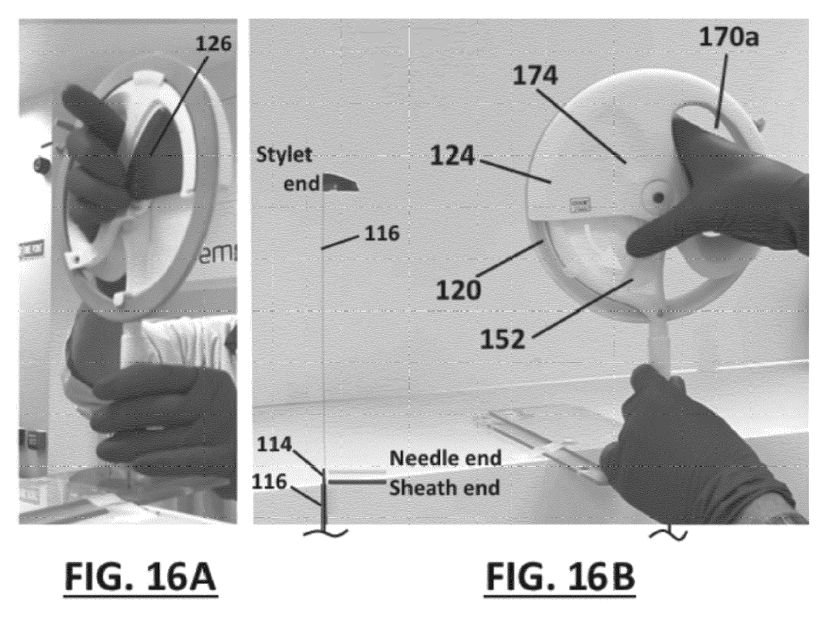

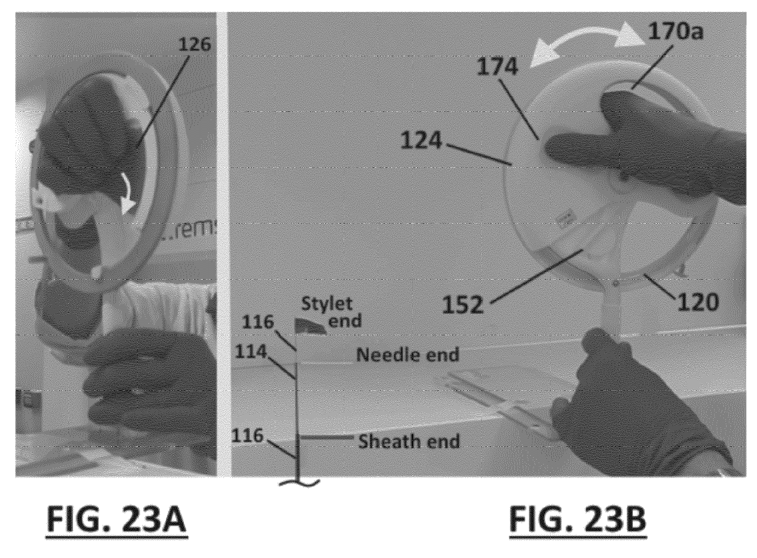

FIGS. 13A-13B show different view perspectives of an embodiment of the device. FIGS. 14-24 show a progression of images portraying actuation of an embodiment of the device to more clearly illustrate how rotation and other actuation of the device effects movement of the sheath 112, needle 114, and/or stylet 116 in one non-limiting illustration of actuation methods for the embodiment depicted. Each A/B pair of FIGS. 15A-23B shows in the left (A) panel a reverse view of the handle end with a user's hand, while the right (B) panel shows a user-perspective view of the device handle and distal end with actuation states through a method of operation as described below. Those of skill in the art will appreciate the functional and structural aspects disclosed thereby with reference to the narrative above and the other drawing figures. FIG. 14 shows an initial demonstration state of the device with the needle 114 extended slightly out of the sheath 112, and the stylet extending (in the particular non-limiting example) about 240 mm beyond the needle's distal end terminus. FIGS. 15A-15B show opposing views in the same initial state/position as FIG. 14, with a user's hand engaging the device from a user-side view in FIG. 15B and an opposed view in FIG. 15A. Each of FIGS. 16A/16B through 23A/23B use the same viewer perspectives for illustration of device operation.

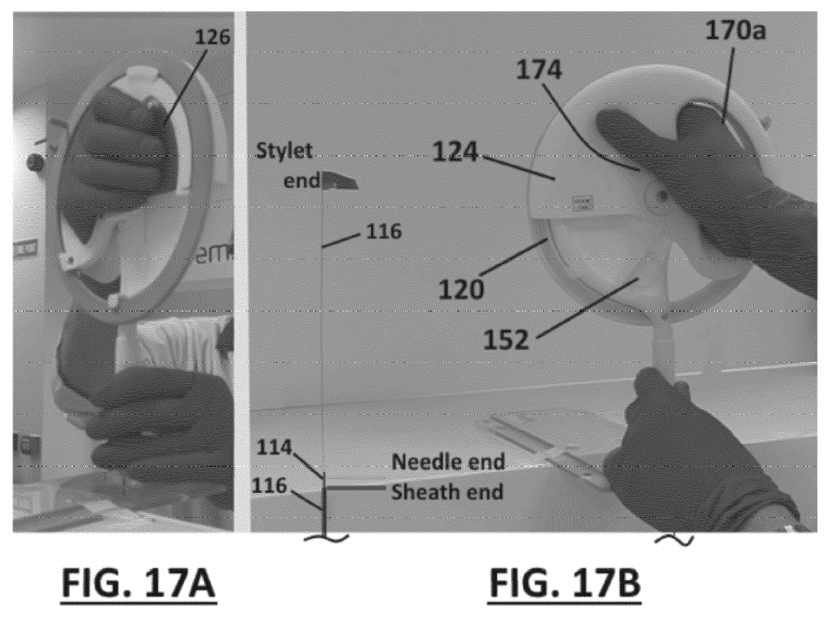

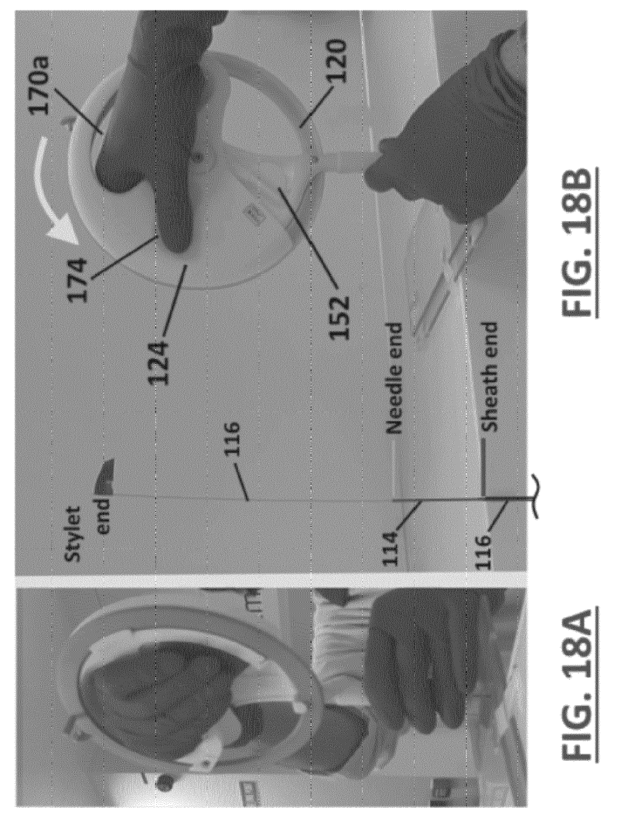

In FIGS. 16A-16B, the user's right thumb has rotated the locking member 152 counterclockwise (in FIG. 16B) from a locked position to an unlocked position (as described above with reference to FIGS. 10B-10C), which does not affect the relative positions of the sheath 112, needle 114, and stylet 116. FIGS. 17A-17B show the user's hand repositioned for the next actuation step, including finger engagement with the stylet retraction lever 126, but with no actuation or other movement of device components. In FIGS. 18A-18B, the user has actuated the device by counterclockwise rotation (in the user's perspective of FIG. 18B), which is shown as having extended the needle 114 and stylet 116 further out of the sheath 112. The sheath distal end terminus remains in place, while the distal needle end terminus moves distally relative to the sheath 112 but little or none relative to the stylet 116. This corresponds with the actuation described above from FIG. 10C to FIG. 10D.

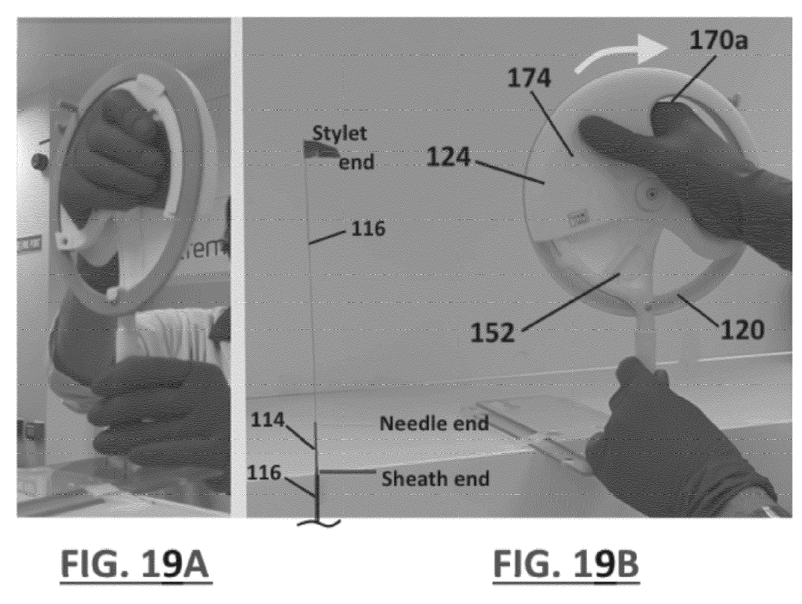

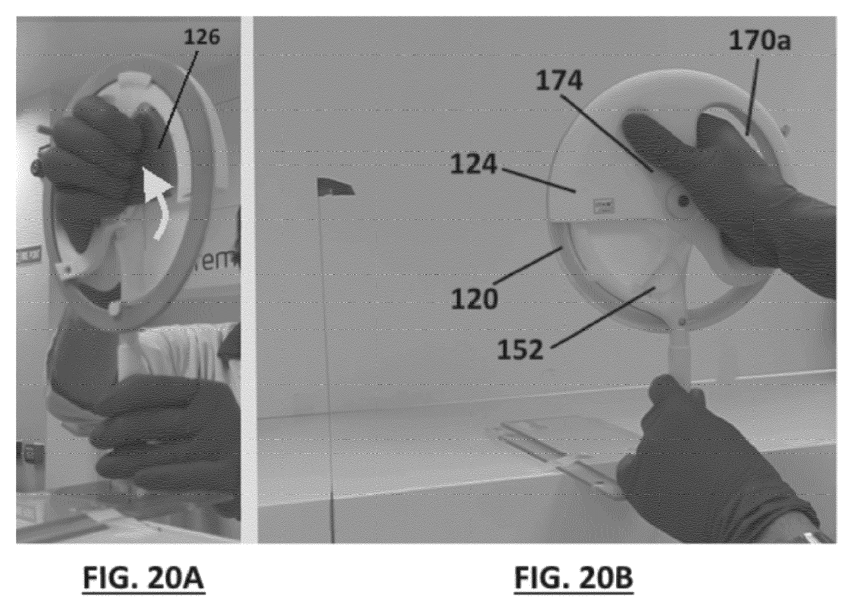

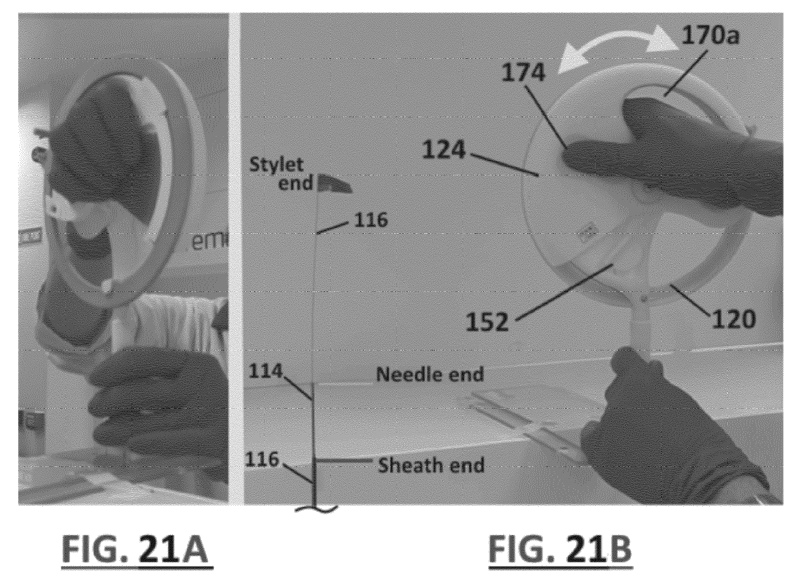

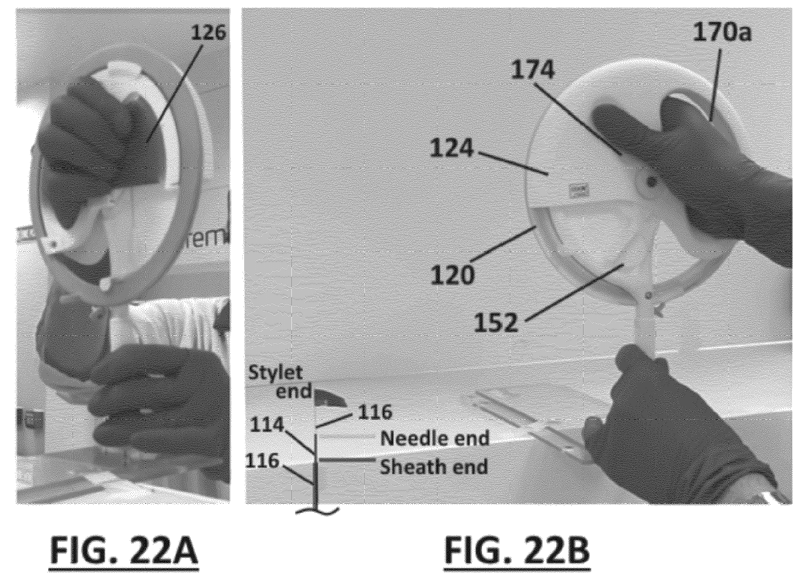

In FIGS. 19A-19B, the user has actuated the device the opposite direction by clockwise rotation (in the user's perspective of FIG. 19B), which is shown as having retracted the needle 114 and stylet 116 back into the sheath 112 by the distance shown differentially from FIG. 18B. The sheath distal end terminus remains in place, while the distal needle end terminus moves proximally relative to the sheath 112 but little or none relative to the stylet 116. This corresponds with the actuation described above from FIG. 10D to FIG. 10E. FIGS. 20A-20B show the user's hand engaging and actuating the stylet retraction lever 126, but with no rotational actuation or other movement of other device components. This matches what is shown and described above with reference to FIGS. 11A and 11B. In FIGS. 21A-21B, the user begins actuating the device with the stylet retraction lever 126 still being engaged by reciprocating/repeated clockwise and counterclockwise rotation (in the user's perspective of FIG. 21B), which is then shown in FIGS. 22A-22B as having retracted the needle 114 and stylet 116 back into the sheath 112 by the distance shown (with the stylet distal end bearing a flag, and the distal terminal ends of the needle and sheath identified by text, as in foregoing figures). This corresponds with the actuation described above from FIG. 11C to FIG. 11E, except that the present illustrative figures 22A-22B do not include the stylet being fully retracted into the needle lumen.

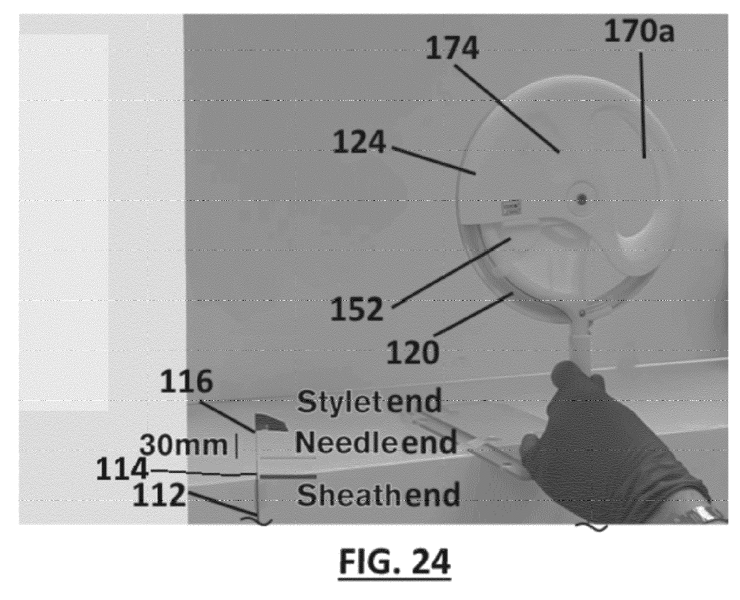

Then, FIGS. 23A-23B show that the user can release the stylet retraction lever 126 (FIG. 23A) and actuate the handle by rotation to again extend the needle and 114 and stylet 116 relative to the sheath 112 from the position shown in FIG. 22B (with the stylet distal end bearing a flag, and the distal terminal ends of the needle and sheath again identified by text). Finally, FIG. 24 shows a contrast with FIG. 14 wherein -through the course of actuation depicted in the intervening drawing figures- a 210 mm length of the stylet has been retracted into the sheath by winding onto the reel (from the 240 mm length identified in FIG. 14 down to the 30 mm length shown in FIG. 24). When used in a patient (e.g., without the stylet-end marker flag), a final state after operation to fully retract the stylet into needle while inside patient will allow biopsy collection by the needle, after which the biopsy sample can be collected by reverse rotation of the stylet reel relative to the outer handle, which will advance the stylet out of the needle, thereby ejecting the sample onto an appropriate surface or into a desire container, medium, or the like. A volume of liquid in the needle proximal of the distal needle end can function as a "liquid stylet" and provide for exerting suction into the needle distal end when that distal needle end is contacting and/or within tissue, thereby providing for sample collection.

Those of skill in the art will appreciate that embodiments not expressly illustrated herein may be practiced within the scope of the claims, including that features described herein for different embodiments may be combined with each other and/or with currently-known or future-developed technologies while remaining within the scope of the claims. Although specific terms are employed herein, they are used in a generic and descriptive sense only and not for purposes of limitation unless specifically defined by context, usage, or other explicit designation. It is therefore intended that the foregoing detailed description be regarded as illustrative rather than limiting. And, it should be understood that the following claims, including all equivalents, are intended to define the spirit and scope of this invention. Furthermore, the advantages described above are not necessarily the only advantages of the invention, and it is not necessarily expected that all of the described advantages will be achieved with every embodiment.

## Claims

1. A needle management device, the needle management device comprising:
a needle extending from a handle; and
a reel coupled to the handle,
wherein the handle is rotatable with respect to the reel to effect longitudinal movement of the needle along a longitudinal axis of the needle, and wherein an axis of rotation of the handle and an axis of rotation of the reel each is not coaxial with respect to the longitudinal axis of the needle.

2. The needle management device of claim 1, further comprising a stylet extending from the reel, wherein the needle at least partially surrounds the stylet, and wherein the reel is rotatable in a manner effecting longitudinal position change of the stylet along a longitudinal axis of the stylet, preferably where the reel is adapted to receive the stylet as the stylet is removed from the needle.

3. The needle management device of claim 2, further comprising a base in mechanical communication with the reel, wherein the needle management device comprises a first state wherein the reel is rotatable in at least two directions with respect to the base, and wherein at least a portion of the stylet is coaxial with the base, preferably where the reel and the handle are rotatable with respect to the base about a common axis.

4. The needle management device of claim 3, further comprising a second state wherein the reel is substantially prevented from rotating in at least one of the at least two directions by an engagement of a ratchet pawl with at least one tooth located on the reel.

5. The needle management device of any preceding claim, further comprising a ratchet mechanism preferably wherein the ratchet mechanism comprises a lever with a cam, the cam configured to contact a cam follower when the lever is in an engaged position.

6. The needle management device of claim 5, wherein the lever is configured to occupy the engaged position or an unengaged position, and wherein the cam follower does not contact the cam when the lever is in the unengaged position.

7. The needle management device of any preceding claim, wherein the needle management device further comprises a ratchet pawl configured to communicate with teeth to limit the rotation of the reel with respect to the handle in at least one direction, preferably wherein the reel comprises said teeth,

8. The needle management device of any preceding claim, configured with stylet management operability and further comprising a stylet extending from the reel, wherein the needle at least partially surrounds the stylet, and wherein the reel is rotatable in a manner effecting longitudinal position change of the stylet along a longitudinal axis of the stylet.

9. The needle management device of claim 8, further comprising a base in mechanical communication with the reel, wherein the needle management device comprises a first state wherein the reel is rotatable in at least two directions with respect to the base, and wherein at least a portion of the stylet is coaxial with the base and preferably further comprising a second state wherein the reel is substantially prevented from rotating in at least one of the at least two directions by an engagement of a ratchet pawl with at least one tooth located on the reel.

10. A needle and stylet management device, the needle and stylet management device comprising:
a reel configured to effect a longitudinal position of a stylet along a longitudinal axis of the stylet;
a handle configured to effect the longitudinal position of a needle along the longitudinal axis of the needle;
a base coupled to the reel;
a first state, wherein the reel is rotatable with respect to the base in at least two directions; and
a second state, wherein rotation of the reel is limited with respect to the base in at least one direction.

11. The needle and stylet management device of claim 10, wherein as the handle rotates in the first state, the handle is configured to effect the rotation of the reel.

12. The needle and stylet management device of claim 10 or 11, further comprising a ratchet mechanism with a ratchet pawl configured to engage and unengaged the reel.

13. The needle and stylet management device of claim 10, 11, or 12, further comprising a locking member coupled to the base and configured to selectively limit the rotation of the handle in at least one direction.

14. The needle and stylet management device of one of claims 10 to 13, wherein the reel and the handle are rotatable with respect to the base about a common axis, preferably wherein the reel is adapted through a spiral path or other means to accommodate the stylet on removal from the needle.

15. The needle and stylet management device of one of claims 10 to 14, further comprising a stylet connected with the reel so as to be wound on or off the reel on rotation of the reel in respective opposite senses and a needle in which the stylet is coaxially received, the needle being connected with the handle.
